# EUROPÄISCHE PATENTSCHRIFT

(11) **EP 0 635 473 B1**
(45) Veröffentlichungstag und Bekanntmachung des Hinweises auf die Patenterteilung: **19.03.1997**
(21) Anmeldenummer: 94110779.9
(22) Anmeldetag: 12.07.1994
(51) Int. Cl.: C07C 51/60, B01J 14/00

(54) **Verfahren zur Herstellung von Carbonsäurechloriden**
Process for the preparation of carboxylic acid chlorides
Procédé pour la préparation de chlorures d'acides carboxyliques

(30) Priorität: 22.07.1993 DE 4324605
(43) Veröffentlichungstag der Anmeldung: 25.01.1995
(73) Patentinhaber: BASF Aktiengesellschaft, 67063 Ludwigshafen (DE)
(72) Erfinder: Ksoll, Dr. Peter, D-53127 Bonn (DE); Reuther, Dr. Wolfgang, D-69118 Heidelberg (DE); Ettl, Dr. Roland, D-67454 Hassloch (DE)

(56) Entgegenhaltungen:
- EP-A- 0 475 137
- US-A- 4 294 800

## Beschreibung

Die vorliegende Erfindung betrifft ein verbessertes Verfahren zur kontinuierlichen Herstellung von Carbonsäurechloriden (I) der allgemeinen Formel R-COCl, in der R für einen C-organischen Rest mit 1 bis 30 C-Atomen steht, durch Umsetzung einer Carbonsäure R-COOH (II) mit einem Umsetzungsprodukt (III) aus Phosgen (IV) und einem N,N-substituierten Formamid (V).

Carbonsäurechloride stellen reaktive und technisch wichtige Ausgangsprodukte für eine Vielzahl von Carbonsäurederivaten wie Estern und Amiden dar. Die Herstellung der Carbonsäurechloride aus Carbonsäuren und Phosgen erfolgt nach der allgemeinen Reaktionsgleichung:

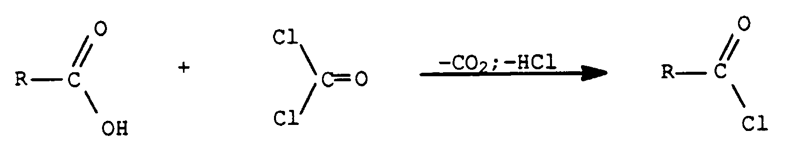

In aller Regel nimmt man diese Reaktion mit Hilfe eines Phosgenüberträgers vor, wobei sich, wie allgemein bekannt ist, N,N-disubstituierte Formamide als besonders geeignet erwiesen haben. Diese Formamide bilden mit dem Phosgen sog. Vilsmeyer-Verbindungen 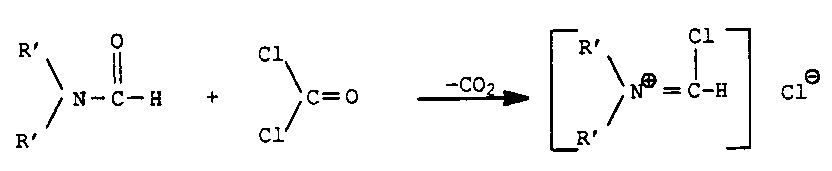 die unter Rückbildung der Formamide als eigentliche Chlorierungsmittel wirken:

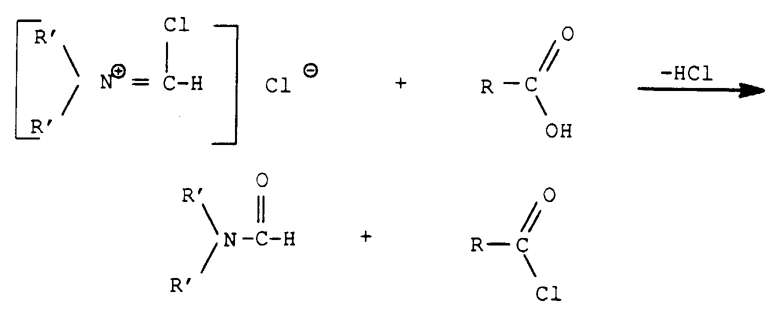

Da sich die Formamide wieder zurückbilden, wirken sie auch in geringen Mengen und werden deshalb auch nicht ganz zutreffend als Katalysatoren bezeichnet.

Aus der EP-A 475 137 ist es speziell bekannt, Carbonsäure und Phosgen in eine stehende Phase aus der Vilsmeyer-Verbindung ("Katalysatoraddukt") einzuleiten und zur Reaktion zu bringen. Als Katalysatoren kommen z. B. Carbonamide, vorzugsweise N-Alkylformamide zum Einsatz. Durch die Wahl des Katalysatorsystems werden der Ablauf der Phosgenierung einer Carbonsäure zum Carbonsäurechlorid sowie die Aufarbeitung des Ansatzes beeinflußt. Bei dem in der EP-A 475 137 beschriebenen Verfahren fällt allerdings ein Abgas an, das vorwiegend aus Kohlendioxid und Chlorwasserstoff besteht und das daneben noch etwas Phosgen enthält (Seite 4, Zeilen 43-44). Da die Rückgewinnung des Chlorwasserstoffs häufig nicht wirtschaftlich ist, neutralisiert man das Abgas mit einer alkalisch wässrigen Lösung, z. B. mit einer Calcium- oder Natriumhydroxidlösung, wobei sich die entsprechenden Chloride bilden, die dann leicht entsorgt werden können. Sind jedoch größere Mengen an Kohlendioxid im Abgas zugegen, entstehen auch die Carbonate, wodurch ein unnötiger Mehrverbrauch am Neutralisationsmittel bedingt wird.

Der Erfindung lag daher die Aufgabe zugrunde, diesem Nachteil abzuhelfen und die kontinuierliche Herstellung von Carbonsäurechloriden aus Carbonsäure und Phosgen insgesamt wirtschaftlicher und verfahrenstechnisch einfacher zu gestalten als bisher.

Demgemäß wurde ein Verfahren zur kontinuierlichen Herstellung von Carbonsäurechloriden (I) der allgemeinen Formel R-COCl, in der R für einen C-organischen Rest mit 1 bis 30 C-Atomen steht, durch Umsetzung einer Carbonsäure (II) mit einem Umsetzungsprodukt (III) aus Phosgen (IV) und einem N,N-disubstituierten Formamid (V) gefunden, welches dadurch gekennzeichnet ist, daß man II und III im Gleichstrom von unten in eine mit III gefüllte Zone eines Reaktors (1) leitet, das Reaktionsgemisch in überwiegend laminarer Strömung aufsteigen läßt, wobei man eine Phasentrennung in eine obere I-Phase und eine untere Phase III' aus überwiegend III und entstandenem Formamid erhält und I aus der oberen Phase sowie Teile der unteren Phase III' nach Maßgabe ihrer Bildung entnimmt.

Der Substituent R hat vorzugsweise folgende Bedeutung:
- eine C₁-C₃₀-Alkylgruppe, insbesondere eine C₈-C₂₂-Alkylgruppe wie 2-Ethylhexyl oder Octadecyl
- eine C₂-C₃₀-Alkenylgruppe, insbesondere eine C₈-C₂₂-Alkyenylgruppe wie Decenyl oder Octadecenyl
- eine C₂-C₃₀-Alkinylgruppe, insbesondere eine C₈-C₂₂-Alkyinylgruppe wie Decinyl oder Octadecinyl
- eine C₃-C₈-Cycloalkylgruppe, insbesondere eine C₅-C₈-Cycloalkylgruppe wie Cyclopentyl oder Cyclohexyl,
- eine C₄-C₈-Cycloalkenylgruppe, insbesondere eine C₅-C₆-Cycloalkenylgruppe wie Cyclopentenyl oder Cyclohexenyl,
- eine C₇-C₁₂-Aralkylgruppe wie Benzyl oder 2-Phenylethyl,
- eine Arylgruppe wie Phenyl, Napht-1-yl oder Naphth-2-yl.

Diese Reste können ihrerseits inerte Substituenten tragen, z.B. Halogene wie Fluor, Chlor oder Brom; Nitro, Cyano, C₁-C₄-Alkoxy, C₁-C₄-Acyl, C₁-C₄-Acyloxy und C₁-C₄-Oxycarbonyl.

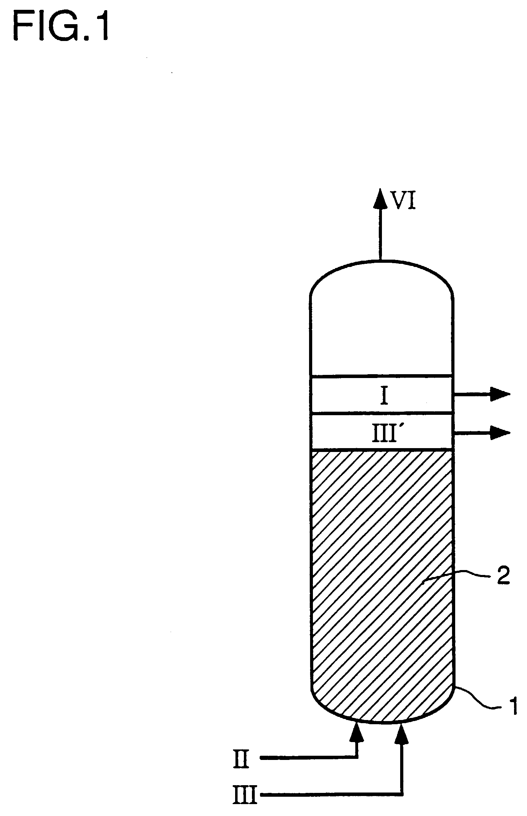

Im folgenden wird das erfindungsgemäße Verfahren anhand von Figur 1 näher beschrieben.

Einem Reaktor (1), der in einer Reaktionszone (2) ein Umsetzungsprodukt (III) aus Phosgen (IV) und einem N,N-disubstituierten Formamid (V) enthält, werden von unten Carbonsäure (II) und Umsetzungsprodukt (III), welches vorzugsweise unmittelbar vorher in einer getrennten Apparatur aus Phosgen und dem Formamid hergestellt wird, im Gleichstrom zugeführt. Bei der Reaktion bildet sich das Carbonsäurechlorid (I), wobei gleichzeitig Chlorwasserstoff (VI) freigesetzt wird und (III) an gebundenem Chlor durch Umsetzung zu Formamid (V) verarmt und sich somit eine Phase (III') ausbildet, die vorwiegend aus (III) sowie dem entstandenem Formamid (V) besteht.

Oberhalb der Reaktionszone sammeln sich die entstehenden Komponenten (I) und (III'), wobei aufgrund der Dichteunterschiede eine Phasentrennung erfolgt. Das in hoher Reinheit anfallende Carbonsäurechlorid (I) sowie die Phase (III') werden als flüssige Seitenströme abgetrennt und der bei der Umsetzung frei werdende Chlorwasserstoff (VI) wird über Kopf abgezogen.

Die Zugabe von (II) und (III) in den Reaktor (1) erfolgt nach Maßgabe der Entnahme von (I) und (III'). Zur Erleichterung der Phasentrennung bemißt man die Strömungsgeschwindigkeit der Carbonsäure (II) und des Umsetzungsproduktes (III) so, daß sich eine praktisch laminare Strömung einstellt.

Das Verfahren wird vorwiegend im Temperaturbereich von 20 bis 140°C, vorzugsweise 30 bis 70°C sowie bei Normaldruck ausgeführt. Verminderter Druck kann sich empfehlen, um die Abtrennung der Spaltgase zu erleichtern, und erhöhter Druck, etwa bis zu 5 bar, kann im Falle leicht flüchtiger Komponenten des Reaktionsgemivon Vorteil sein.

Geeignete Reaktoren sind alle diejenigen, in denen die Umsetzung ohne nennenswerte Rückvermischung nach dem Rohrreaktorprinzip erfolgt, also z. B. stehende Rohrreaktoren oder Rohre in Spiralform, an welche sich die Vorrichtung für die Phasentrennung anschließt. Vor Eintritt in die laminar durchströmte Reaktionszone kann auch eine kurze Durchmischung der Reaktionspartner (II) und (III) erfolgen.

Zweckmäßigerweise wird bei dem Verfahren ein Überschuß an Phase (III) gegenüber der Carbonsäure (II) eingesetzt, jedoch sind auch äquimolare Mengen möglich. Das Reaktionsabgas enthält nur unwesentliche Reste an Phosgen, dessen Anteil in der Regel zwischen etwa 0,001 und 0,1 Vol% liegt.

Das Verhältnis von Höhe zu Durchmesser des Reaktors liegt in einem Bereich von 5:1 bis 200:1, besonders bevorzugt zwischen 10:1 bis 50:1.

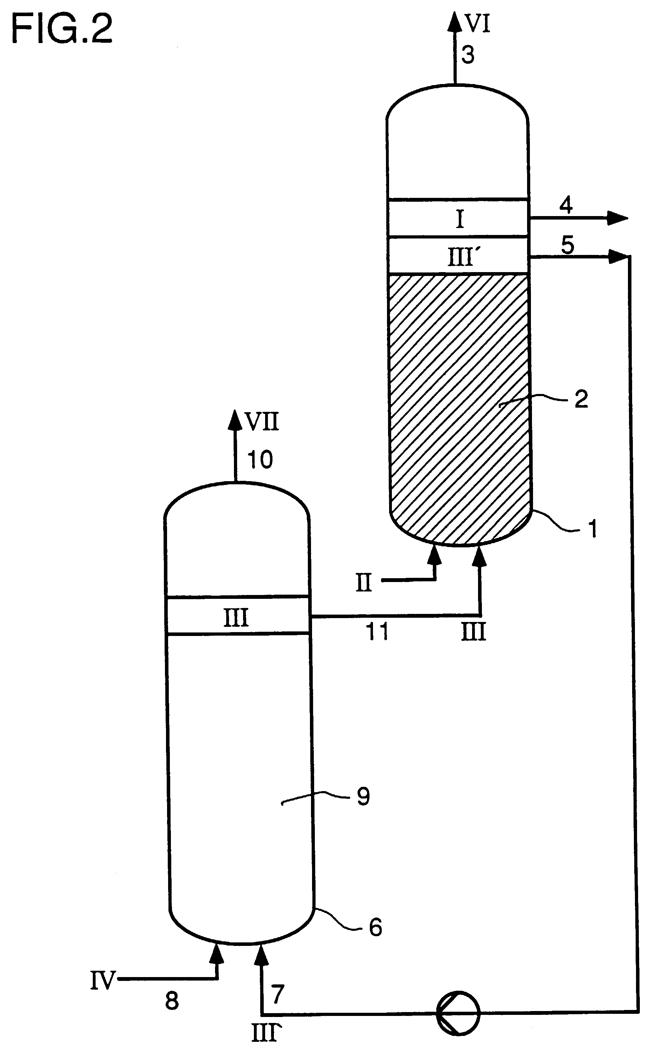

Anhand von Figur 2 wird eine besonders bevorzugte Ausführungsform der Erfindung unter Einbeziehung der Herstellung des Umsetzungsproduktes (III) näher beschrieben.

Analog zu dem in Figur 1 geschilderten Verfahren erfolgt in der Reaktionszone (2) des Reaktors (1) die Bildung des Carbonsäurechlorids (I) aus der Carbonsäure (II) und dem Umsetzungsprodukt (III), wobei der freiwerdende Chlorwasserstoff (VI) am Kopf des Reaktors über die Leitung (3) abgezogen wird. Das gebildete Carbonsäurechlorid (I) wird über die Leitung (4) abgeführt.

Das Umsetzungsprodukt (III') wird über Leitung (5) einem zweiten Reaktor (6) von unten über den Einlaß (7) zugeführt. Mittels Leitung (8) wird Phosgen (IV) von unten in den Reaktor (6) gegeben. In der Reaktionszone (9) des Reaktors (6) erfolgt die Umsetzung des Phosgens (IV) mit dem Formamid (V), und es bildet sich wieder das Umsetzungsprodukt (III) unter Freisetzung von Kohlendioxid (VII), das über Leitung (10) aus dem Reaktor (6) abgeführt wird. Das gewonnene Umsetzungsprodukt (III) wird mittels Leitung (11) erneut in den Reaktor (1) eingebracht.

Zur Verbesserung der Abtrennung der innerhalb des Reaktors (1) gebildeten Produkte Carbonsäurechlorid (I) und Umsetzungsprodukt (III') können diese Stoffe gemeinsam einem separaten Abscheidebehälter zugeführt werden, in dem die einzelnen Komponenten in hoher Reinheit anfallen.

Mittels Leitfähigkeitsmeßsonden, die beispielsweise induktiv messen, kann der Gehalt an Vilsmeyer-Verbindung ermittelt werden. Ein Anteil von etwa 50 bis 80 % wird besonders bevorzugt.

Die Zirkulation zwischen den beiden Reaktoren wird durch die Gasbildung einerseits und durch die Dichteerniedrigung des entstehenden Carbonsäurechlorids erzeugt. Sie kann bei Bedarf durch Pumpen unterstützt werden.

Zweckmäßigerweise werden bei dem Verfahren im wesentlichen äquimolare Mengen an Carbonsäure (II) und Phosgen (IV) eingesetzt.

Bei Bedarf kann dem Reaktionsgemisch ein inertes Lösungsmittel wie z. B. gesättigte aliphatische Kohlenwasserstoffe, Ether, Acetonitril, Benzol, Toluol oder Cyclohexan zugesetzt werden. Dies ist z. B. beim Einsatz fester Säuren oder zur Verbesserung der Phasentrennung zweckmäßig.

Als N,N-Dialkylformamide eignen sich insbesondere Verbindungen mit bis zu insgesamt 12 C-Atomen in den Alkylresten, die gleich oder verschieden sein können oder auch zu einem 5- bis 7-gliedrigen Ring verbunden sein können.

Das erfindungsgemäße Verfahren bietet eine einfache und kostengünstige Möglichkeit, Carbonsäurechloride kontinuierlich von hoher Reinheit herzustellen. Die bei der Gewinnung von Carbonsäurechloriden anfallenden Abgase Chlorwasserstoff (VI) und Kohlendioxid (VII) entstehen getrennt und können getrennt entsorgt werden.

### Beispiel

### Herstellung von Stearinsäurechlorid

In einem Laborreaktor von 160 cm Höhe und 10 cm Durchmesser wurde eine Katalysatorlösung bestehend aus einem Umsetzungsprodukt von Diethylformamid und Phosgen vorgelegt. Der Anteil an aktivierter Vilsmeyerverbindung in der Katalysatorlösung betrug ca. 75 Gew.-%. Anschließend wurden bei ca. 60°C, je 3 mol/h, Stearinsäure zugegeben.

Das Stearinsäurechlorid trennte sich als spezifisch leichtere Phase am Kopf des Reaktors ab und wurde abgezogen. Im gewonnenen Produkt waren 99,7 Gew.-% an Säurechlorid enthalten; der Anteil an nicht umgesetzter Carbonsäure war kleiner als 0,1 Gew.-%. Das Produkt war klar und hatte eine Jodfarbzahl von 5.

## Patentansprüche

1. Verfahren zur kontinuierlichen Herstellung von Carbonsäurechloriden (I) der allgemeinen Formel R-COCl, in der R für einen C-organischen Rest mit 1 bis 30 C-Atomen steht, durch Umsetzung einer Carbonsäure R-COOH (II) mit einem Umsetzungsprodukt (III) aus Phosgen (IV) und einem N,N-disubstituierten Formamid (V), dadurch gekennzeichnet, daß man II und III im Gleichstrom von unten in eine mit III gefüllte Zone (2) eines Reaktors (1) leitet, das Reaktionsgemisch in überwiegend laminarer Strömung aufsteigen läßt, wobei man eine Phasentrennung in eine obere Phase I und eine untere Phase III' aus überwiegend III und entstandenem Formamid erhält und I aus der oberen Phase sowie Teile der unteren Phase III' nach Maßgabe ihrer Bildung entnimmt.

2. Verfahren nach Anspruch 1, dadurch gekennzeichnet, daß man die in dem Reaktor (1) gebildete Phase III' zusammen mit Phosgen (IV) einem zweiten Reaktor (6) im Gleichstrom zuführt, und das erhaltene Umsetzungsprodukt (III) erneut in den Reaktor (1) einleitet.

## Claims

1. A process for the continuous preparation of carboxylic acid chlorides (I) of the general formula R-COCl, where R is an organic radical having from 1 to 30 carbon atoms, by reacting a carboxylic acid R-COOH (II) with a reaction product (III) of phosgene (IV) and an N,N-disubstituted formamide (V), wherein II and III are passed in cocurrent from below into a III-filled zone (2) of a reactor (1) which allows the reaction mixture to ascend in predominantly laminar flow, with a phase separation into an upper phase I and a lower phase III' comprising predominantly III and formamide formed being obtained and I being taken from the upper phase and parts of the lower phase III' commensurably with their formation.

2. A process as claimed in claim 1, wherein the phase III' formed in the reactor (1) together with phosgene (IV) is fed to a second reactor (6) in cocurrent and the reaction product (III) obtained is reintroduced into the reactor (1).

## Revendications

1. Procédé de préparation continue de chlorure d'acide carboxylique (I) de la formule générale R-COCl, dans laquelle R est mis pour un reste organique en C de 1 à 30 atomes C, par réaction d'un acide carboxylique R-COOH (II) avec un produit de réaction (III) de phosgène (IV) et d'un formamide disubstitué sur N-N (V), caractérisé par le fait que l'on dirige II et III en courant continu, d'en dessous, dans une zone (2) remplie de III, d'un réacteur (1), qu'on laisse remonter le mélange de réaction en courant majoritairement laminaire, en obtenant ainsi une séparation de phases en une phase supérieure (I) et une phase inférieure (III') de majoritairement III et formamide naissant et en prélevant I de la phase supérieure ainsi que de parties de la phase inférieure III' en proportion de leur formation.

2. Procédé selon la revendication 1, caractérisé par le fait que l'on amène en courant continu dans un deuxième réacteur (6) la phase III formée dans le réacteur (1) ensemble avec du phosgène dans un deuxième réacteur (6) et on introduit à nouveau dans le réacteur (I) le produit de réaction obtenu (III).
